# EUROPÄISCHE PATENTSCHRIFT

(11) **EP 2 150 294 B1**
(45) Veröffentlichungstag und Bekanntmachung des Hinweises auf die Patenterteilung: **19.09.2012**
(21) Anmeldenummer: 08760505.1
(22) Anmeldetag: 04.06.2008
(51) Int. Cl.: A61M 1/36, A61M 1/02, B04B 5/04

(54) **VERFAHREN ZUR ZELLGEWINNUNG**
METHOD FOR CELL EXTRACTION
PROCÉDÉ D'OBTENTION DE CELLULES

(30) Priorität: 05.06.2007 DE 102007000309
(43) Veröffentlichungstag der Anmeldung: 10.02.2010
(73) Patentinhaber: Terumo Europe NV, 3001 Leuven (BE); Andreas Hettich GmbH & Co. KG, 78532 Tuttlingen (DE)
(72) Erfinder: EBERLE, Klaus-Günter, 78532 Tuttlingen (DE); BISET, Roland, 3001 Leuven (BE)
(74) Vertreter: TBK
(86) Internationale Anmeldenummer: PCT/EP2008/056925
(87) Internationale Veröffentlichungsnummer: WO 2008/148810

(56) Entgegenhaltungen:
- EP-A- 1 512 464
- WO-A-02/053292
- WO-A-03/089027
- GB-A- 2 174 149
- US-A- 5 734 464

## Beschreibung

### Technisches Gebiet

Die Erfindung betrifft ein Verfahren zur Zellgewinnung aus Vollblut oder aus einem Bluterzeugnis, einen zur Ausführung des Verfahrens geeigneten Einsatz und eine entsprechende Zentrifuge.

### Stand der Technik

In der Transfusionsmedizin hat sich seit dem Beginn der neunziger Jahre die so genannte Blutkomponententherapie durchgesetzt. Dies bedeutet, dass einem Patienten anstelle einer Vollblutkonserve lediglich diejenigen Blutbestandteile verabreicht werden, die der einzelne Patient benötigt. Durch diese getrennte Verabreichung der einzelnen Blutbestandteile ist es möglich, mit einer einzigen Blutkonserve durchschnittlich 1,8 Patienten optimal zu helfen.

Die wesentlichen Blutbestandteile sind
die roten Blutkörperchen im so genannten Erythrozytenkonzentrat, die zur Aufrechterhaltung der Sauerstoffversorgung nach schweren Blutverlusten transfundiert werden,
die Blutplättchen im Thrombozytenkonzentrat, die bei Gerinnungsstörungen (Bluterkrankheit) verabreicht werden, und
das Blutplasma, das bei Gerinnungsstörungen und Volumendefiziten verabreicht wird. Abgesehen davon ist Blutplasma ein wesentlicher Grundbestandteil zur Herstellung vieler Medikamente.

Das Auftrennen der einzelnen Blutbestandteile, das als Zellgewinnung bezeichnet wird, erfolgt bekannt durch ein Behandeln des Bluts in einer Zentrifuge. Durch das Zentrifugieren werden die einzelnen Blutbestandteile voneinander getrennt und können dann einzeln in entsprechende Behältnisse abgefüllt und verwendet werden.

Beispielsweise ist aus der EP 1 351 772 B1 eine derartige Zentrifuge bekannt. Gemäß diesem Stand der Technik sind in einem Rotor einer Zentrifuge eine Vielzahl von Einsätzen um eine Nabe angeordnet. Die Einsätze sind dabei fest in dem Rotor gehalten, sodaß die Blutbeutel stehend zentrifugiert werden. Die Einsätze weisen in Ihrem Inneren

Aufnahmen für einen Vollblut enthaltenden Blutbeutel und für Produktbeutel auf, in denen das Plasma bzw. das Erythrozytenkonzentrat gesammelt wird. Um zu verhindern, dass es nach erfolgter Trennung der einzelnen Bestandteile zu einem Nachfließen und abermaligen Vermischen der Produkte kommt, sind in dem Einsatz verschiedene Klemmeinrichtungen vorgesehen, mit denen die einzelnen Schläuche abgeklemmt werden können. Zum Entnehmen der Beutel aus dem Einsatz nach erfolgter Trennung, müssen die einzelne Anschlusschläuche der Beutel durch geeignete Maßnahmen verschlossen werden. Erst danach können die Klemmen des Einsatzes geöffnet, die Beutel entnommen und der Einsatz zur Aufnahme eines neuen Beutelsatzes vorbereitet werden.

Nach der Trennung und dem Abfüllen des Plasmas bzw. der roten Blutkörperchen verbleibt ein als "buffy coat" bezeichnetes Gemisch in den Blutbeuteln. Dieses "buffy coat" besteht im Wesentlichen aus Blutplättchen sowie weißen und roten Blutkörperchen. Zur Gewinnung der Blutplättchen aus diesem "buffy coat" wird dieses mit einer Additivlösung verdünnt und dieses verdünnte "buffy coat" durch Zentrifugieren abermals in seine Bestandteile aufgetrennt.

Aus der WO 03/089027 ist ein System und Verfahren zu diesem Zweck bekannt. Diese Druckschrift offenbart eine Zentrifuge, in deren einzelner Kammer ein ringförmiger Beutel mit einem Gemisch aus "buffy coat" und Additivlösung eingelegt werden. Die Blutbestandteile werden dann durch einen Zentrifugiervorgang aufgetrennt und die getrennten Bestandteile durch eine Schlauchleitung über einen in dem Bereich der Nabe angeordneten Filter zu einem ebenfalls in dem Bereich der Nabe angeordneten Sammelbehälter geleitet.

Die Druckschrift WO 02/053292 A betrifft eine Zentrifuge mit Blutbeutelsystem mit oberem und unterem Abgang zur Trennung von Blutkomponenten, welche Zentrifuge einen um eine Nabe rotierend antreibbaren Rotor beinhaltet, und in diesem Blutbeutelsystem mindestens ein herkömmlicher Vollblutbeutel mit oberem und unterem Abgang stehend anordenbar ist, wobei der Vollblutbeutel derart am Rotor anordenbar ist, dass der eine Abgang des Vollblutbeutels in der einen radialen Richtung geknickt ist und der gegenüberliegende Abgang des Vollblutbeutels in der anderen radialen Richtung geknickt ist, so dass insgesamt der Vollblutbeutel in seiner Funktionslage mit seinen Abgängen eine etwa Z- oder S-Form einnimmt. Der obere Abgang des Vollblutbeutels liegt dabei radial nach innen und der untere Abgang radial nach außen. Der Vollblutbeutel wird in einem Einsatz gehalten, der wiederum in einer etwa sektorförmigen Kassette gehalten wird, so dass mehrere derartige Kassetten gleichmäßig am Umfang verteilt am Rotor angeordnet sind.

Aus der Druckschrift GB 2 174 149 A ist eine Vorrichtung bekannt, die eine Vielzahl von Elementen aufweist, um ein Blutprodukt aus einem flexiblen Beutel durch einen Schlauch zu einem anderen Beutel zu übertragen. Der flexible Beutel wird durch eine schwenkbare Platte 32 schrittweise zusammengedrückt, während das jeweilige Element schrittweise vorangeführt wird. Nach einer vorbestimmten Anzahl von Schritten ist die Übertragung des Blutbestandteils beendet. Der Schlauch wird abgeschnitten und dabei abgedichtet.

Aus der Druckschrift EP 1 512 464 A und aus der Druckschrift US 5 734 464 A sind weitere Vorrichtungen bzw. Verfahren bekannt, um Blutbestandteile aus einem flexiblen Beutel in einen anderen Beutel zu übertragen.

### Darstellung der Erfindung

### Technische Aufgabe

Es ist die Aufgabe der Erfindung, ein verbessertes Verfahren zur Zellgewinnung aus Blut oder aus einem Bluterzeugnis bereitzustellen, das eine verbesserte Ausbeute bei der Zellgewinnung und eine wirtschaftlichere Zellgewinnung ermöglicht.

### Technische Lösung

Die Aufgabe der Erfindung wird durch Verfahren zur Zellgewinnung nach Anspruch 1, einen Einsatz nach Anspruch 10 und eine Zentrifuge nach Anspruch 18 gelöst. Vorteilhafte Ausführungsformen der Erfindung werden gemäß den abhängigen Ansprüchen gelöst.

Bei dem Verfahren zur Zellgewinnung aus Blut oder aus Bluterzeugnissen gemäß der Erfindung wird ein Blutbeutel mit einem Blutprodukt in einen Blutbeutelbereich eines Einsatzes eingelegt. Danach wird ein mit dem Blutbeutel verbundener Schlauch in einen Produkt-Förderweg eingelegt. Der Produkt-Förderweg verbindet den Blutbeutel mit einem Produktbeutel. Entlang des Produkt-Förderwegs sind ein erster und ein zweiter Sensor positioniert. Als nächstes wird der Einsatz in den Rotor einer Zentrifuge eingesetzt.

Danach wird die Zentrifuge rotiert, um die einzelnen Blutbestandteile aufzutrennen. Dieser Schritt wird gefolgt durch ein Fördern einer vorbestimmten Menge eines Blutbestandteils entlang des Produkt-Förderwegs mit einer ersten Strömungsgeschwindigkeit. Als nächstes kann die Strömungsgeschwindigkeit reduziert werden, und der Blutbestandteil weiter gefördert werden. Dabei bleibt auch die Rotation der Zentrifuge aktiv.

Der erste Sensor erfasst während des Förderns die Zusammensetzung des Blutbestandteils. Bevorzugt erfolgt dies aber erst nach der möglichen Reduzierung der ersten Strömungsgeschwindigkeit.

Bei einer vorbestimmten Zusammensetzung des Blutbestandteils gibt der erste Sensor ein entsprechendes Signal ab. Bevorzugt wird der zweite Sensor dabei erst nach der Abgabe des Signals durch den ersten Sensors aktiviert. Der zweite Sensor erfasst dann die Zusammensetzung des Blutbestandteils und gibt ein End-Signal ab, wenn er ebenfalls die vorbestimmte Zusammensetzung erfasst. Damit wird das Fördern beendet. Alternativ zu der Beendigung durch den zweiten Sensor kann das Fördern auch nach dem Verstreichen eines bestimmten Zeitraums seit der Abgabe des Signals von dem ersten Sensor beendet werden. Mit der Beendigung des Förderns wird auch das Rotieren beendet.

Unter der vorbestimmten Zusammensetzung des Blutbestandteils ist das zu gewinnende Blutprodukt gemeint, das einen vorbestimmten Anteil an einem nicht erwünschten Blutprodukt aufweist. Ist in dem Bereich des Sensors der vorbestimmte Anteil an dem nicht erwünschten Blutprodukt erreicht, entspricht dies der vorbestimmten Zusammensetzung.

Durch vorteilhaftes Anordnen der Sensoren ist es somit möglich, eine optimale Menge des erwünschten Blutprodukts zu gewinnen, ohne dass ein Anteil des erwünschten Blutprodukts in dem zur Förderung benutzten Schlauch verbleibt.

Vorteilhaft kann vor dem Fördern der vorbestimmten Menge des Blutbestandteils entlang des Produkt-Förderwegs mit einer ersten Strömungsgeschwindigkeit der Blutbestandteil mit einer reduzierten Anfangsgeschwindigkeit gefördert werden. Dies dient dazu, einen in dem Produkt-Förderweg zwischen dem ersten Sensor und dem zweiten Sensor vorgesehenen Filter zu entlüften. Durch die reduzierte Anfangsgeschwindigkeit, d.h. eine langsame Strömungsgeschwindigkeit, ist sichergestellt, dass Luftblasen oder andere Einschlüsse, die den zweiten Sensor eventuell zur Abgabe eines fehlerhaften Signals bringen könnten, nicht in dem Filter verbleiben.

Vorteilhaft wird der Blutbestandteil (Produkt) dazu radial von außen und von unten in den Filter geleitet. Dadurch findet die Filtrierung gegen die Fliehkraft bzw. Zentrifugalkraft statt.

Vor dem Fördern mit der ersten Strömungsgeschwindigkeit bzw. mit der reduzierten Anfangsgeschwindigkeit kann eine auf dem Schlauch vorgesehene Schlauchklemme durch eine in dem Einsatz vorgesehene Betätigungseinrichtung geöffnet werden. Die geschlossene Schlauchklemme stellt während des Hantierens mit dem Blutbeutel sicher, dass der Inhalt vor dem Beginn der Zellgewinnung nicht in den Schlauch eindringt.

Vorteilhaft kann eine Schlauchklemme alternativ oder zusätzlich zwischen dem zweiten Sensor und dem Produktbeutel vorgesehen sein und bei dem Beenden des Förderns geschlossen werden. Dadurch ist eine Beendigung der Förderung zum optimalen Zeitpunkt sichergestellt. Die jeweiligen Schlauchklemmen werden dann beim Beginn der Förderung durch die Betätigungseinrichtungen geöffnet und bei dem Ende der Förderung durch die Betätigungseinrichtung geschlossen.

Die ersten und zweiten Sensoren können vorteilhaft als optische Sensoren ausgebildet sein. Die vorbestimmte Zusammensetzung des geförderten Blutbestandteils kann dabei einen bestimmten Anteil roter Blutkörperchen aufweisen, der von den Sensoren erfasst wird.

Zur Anpassung der Strömungsgeschwindigkeit kann mittels eines bei dem Rotor vorgesehenen Druckelements ein radial nach außen wirkender Druck auf den Blutbeutel ausgeübt werden.

Der erfindungsgemäße Einsatz und die Zentrifuge sind einerseits für die Gewinnung von Zellen und Plasma aus Vollblut geeignet, sind aber auch für die Gewinnung von Zellen aus dem nach einem bekannten Zentrifugiervorgang verbliebenen "buffy coat" vorgesehen.

Dazu wird das "buffy coat" aus mehreren Blutbeuteln gemeinsam mit einer Additivlösung in einem neuen Blutbeutel gesammelt und vermischt. Der neue Blutbeutel entspricht dabei dem Blutbeutel gemäß der Erfindung. Der neue Blutbeutel kann vorteilhaft mit einem Schlauch und/oder einem insbesondere zum Filtern von Leukozyten vorgesehenen Filter vorgesehen sein.

### Kurze Beschreibung der Abbildungen der Zeichnungen

Im Folgenden wird ein Ausführungsbeispiel der Erfindung anhand der Figuren beschrieben. Dabei zeigen:

Fig. 1 eine Draufsicht des erfindungsgemäßen Einsatzes,

Fig. 2 eine perspektivische Ansicht des Einsatzes,

Fig. 3 eine entlang einer Symmetrielinie geschnittene, perspektivische Ansicht des Einsatzes,

Fig. 4 eine die Ansicht der Fig. 4 ergänzende geschnittene, perspektivische Ansicht des Einsatzes,

Fig. 5 eine weitere perspektivische und geschnittene Ansicht des Einsatzes, Einsatzes,

Fig. 6 eine Unterseite einer Abdeckung des Einsatzes,

Fig. 7 eine perspektivische Ansicht eines Aufnahmekastens, und

Fig. 8a bis 8c schematische Schnittansichten des Einsatzes, aus denen die Zellgewinnung ersichtlich ist, und

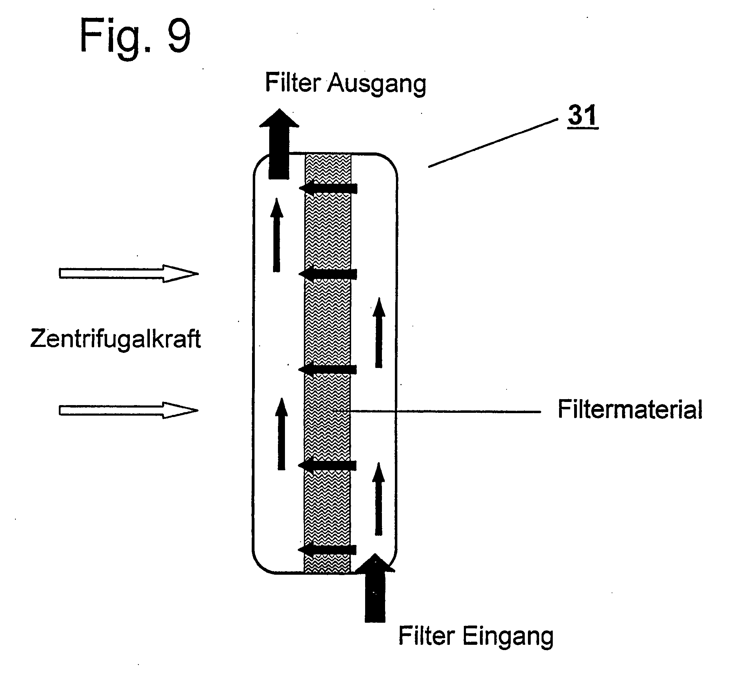

Fig. 9 zeigt einen Strom des Blutprodukts durch den Filter.

### Weg(e) zur Ausführung der Erfindung

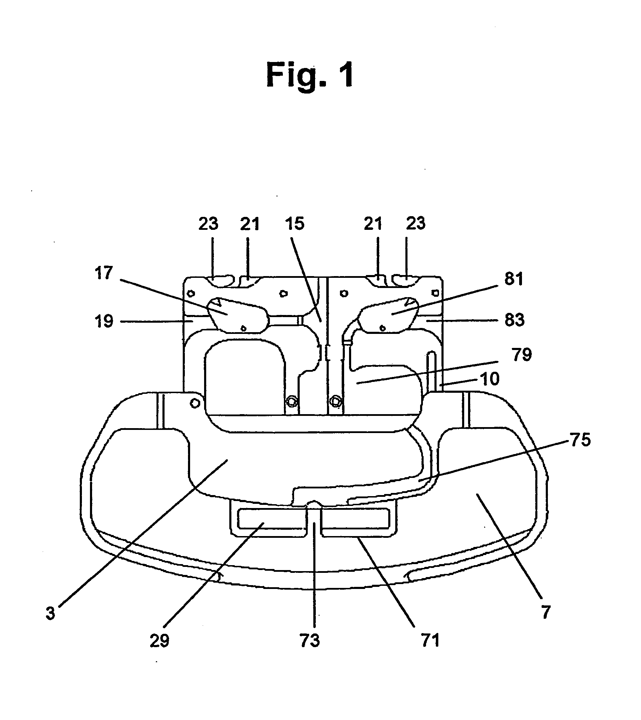

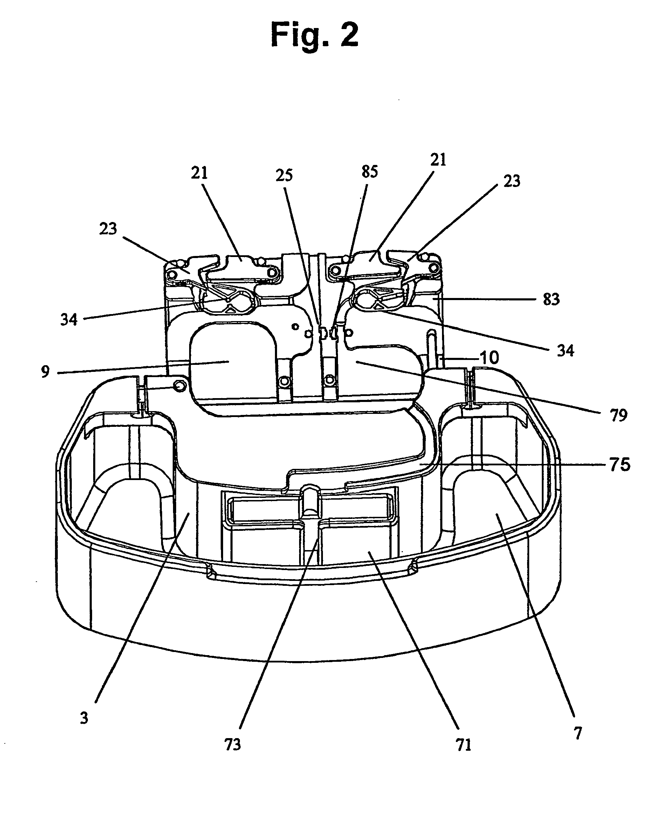

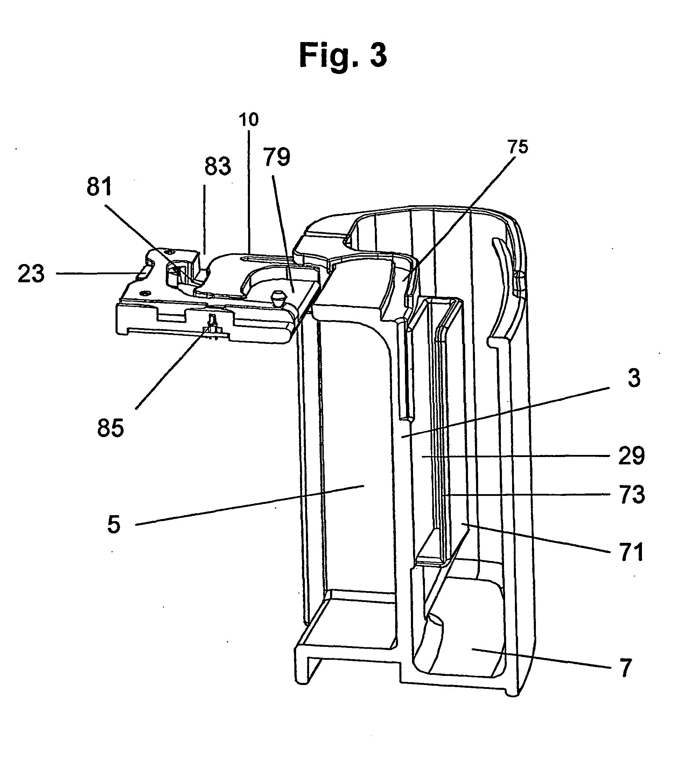

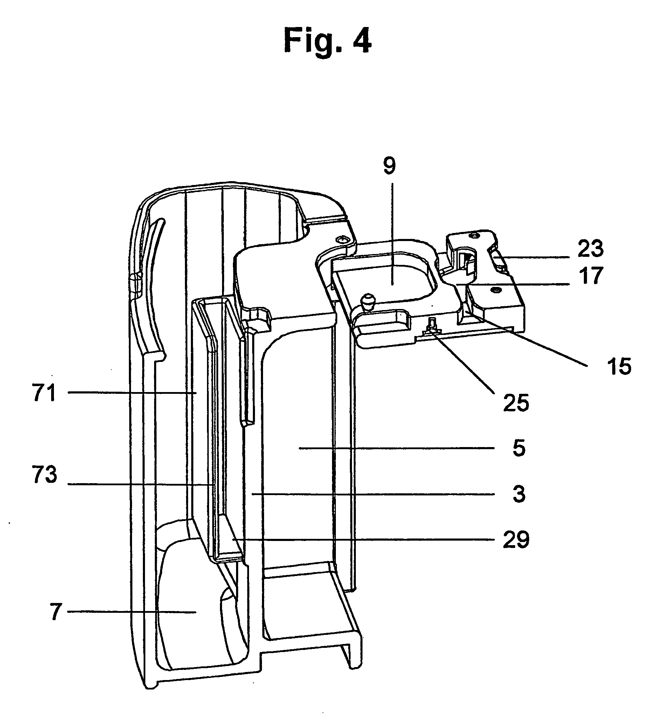

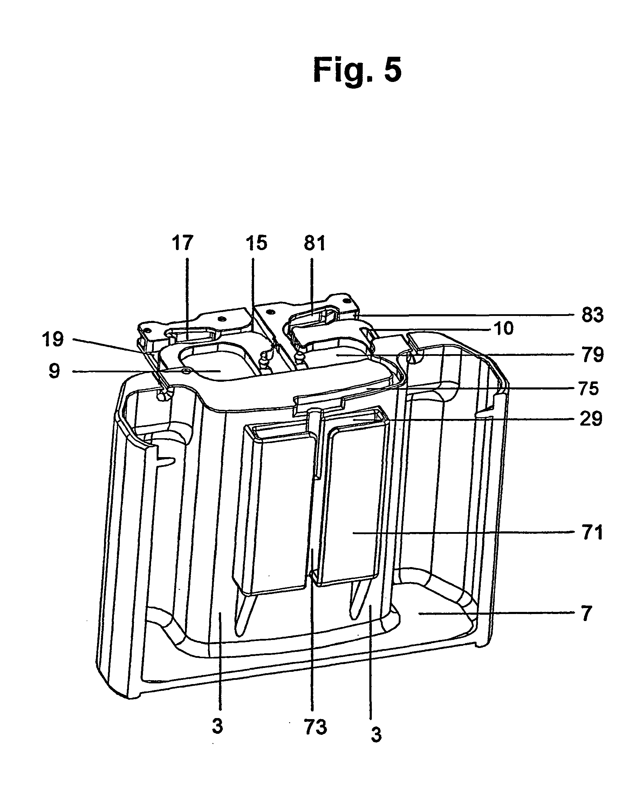

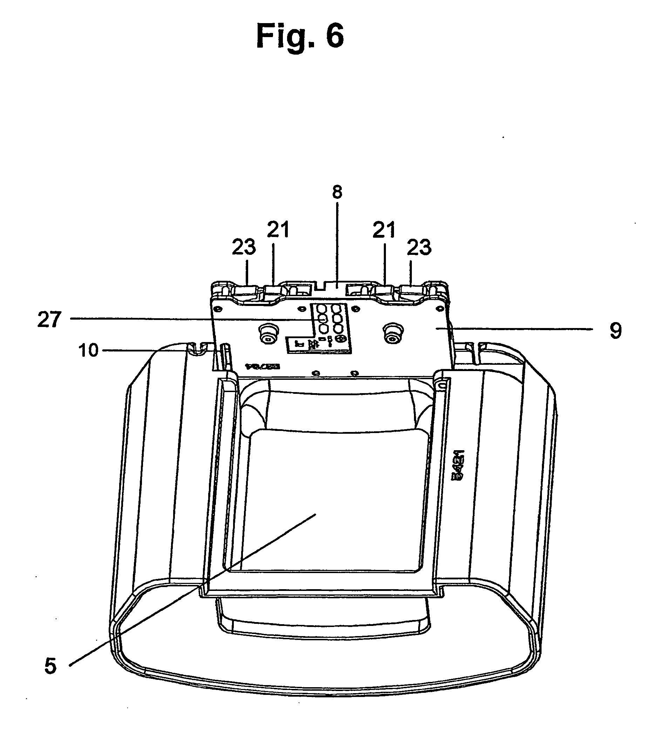

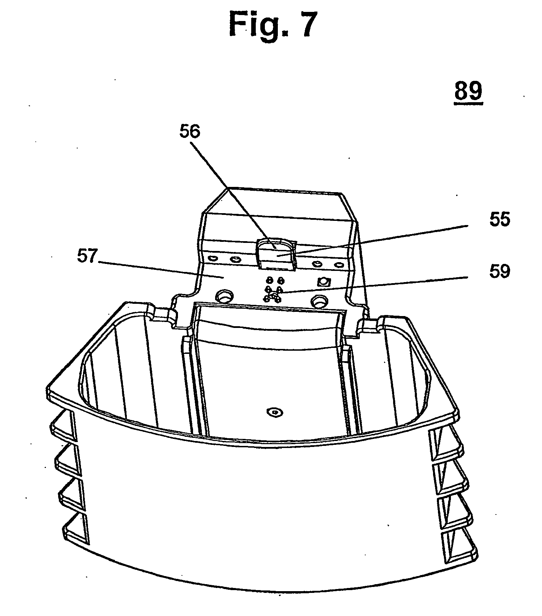

Ein Ausführungsbeispiel der Erfindung wird anhand der Figuren 1 bis 8 beschrieben.

Ein Einsatz y besteht im Wesentlichen aus einer Zwischenwand 3 und einer Abdeckung 9. Die Zwischenwand definiert einen Blutbeutelbereich 5 und einen Produktbeutelbereich 7. Ist der Einsatz y in eine Systembox 89 des Rotors einer Zentrifuge eingeschoben, liegt der Blutbeutelbereich 5 radial innerhalb von der Zwischenwand 3, während der Produktbeutelbereich 7 radial außerhalb von der Zwischenwand 3 liegt. Als Systembox 89 wird dabei ein Aufnahmekasten 89 gemäß der Erfindung bezeichnet.

Über dem Blutbeutelbereich 5 ist eine Abdeckung 9 vorgesehen. Diese ist im Wesentlichen rechteckig ausgebildet und in einem geschlossenen Zustand mit einer Längsseite mit der Zwischenwand 3 in Berührung. An einem Eckpunkt ist die Abdeckung drehbar in der Zwischenwand gelagert, während sie an einem zweiten Eckpunkt mittels einem Riegel 10 mit der Zwischenwand 3 in Eingriff gerät. Zum Öffnen der Abdeckung wird auf den Riegel 10 ein Druck ausgeübt, und danach die Abdeckung zur Seite gedreht. Dadurch ist der Blutbeutelbereich 5 frei zugänglich und kann mit einem Blutbeutel 35 befüllt werden.

Durch den einfachen Drehmechanismus kann ein Schlauch 36 und ein Blutbeutel 35 beim Schließen der Abdeckung 9 einfach und mit einem geringen Aufwand in einer vorgesehenen Lage gehalten und durch Schließen der Abdeckung 9 in dieser vorgesehenen Lage fixiert werden.

Nach dem Schließen der Abdeckung 9 ist es möglich, den Schlauch 36 in Aussparungen 15, 19 einzulegen, die an der Oberseite der Abdeckung 9 ausgebildet sind. Ein erster lichtempfindlicher Sensor 25 ist in der Aussparung 15 vorgesehen.

Eine mit dem Blutbeutel mitgelieferte, auf dem Schlauch vorhandene Schlauchklemme 34 in geschlossenem Zustand, beispielsweise eine von der Firma "Halkey Roberts" hergestellte, wird in einer ebenfalls an der Oberseite der Abdeckung 9 ausgebildeten Aussparung 17 aufgenommen.

Das von dem Blutbeutel 35 aus gesehene ferne Ende des Schlauchs 36 wird zu dem Produktbeutelbereich 7 geführt und ist dort mit einem Leukozytenfilter 31 verbunden, der in einer Aufnahme 29 aufgenommen ist. Dabei ist der Schlauch 36 radial von außen und von unten in den Leukozytenfilter 31 eingeführt. Das Einlegen von Filter 31 und Schlauch 36 wird durch einen Schlitz 73 in einer Außenwand 71 der Aufnahme 29 ermöglicht. Durch den Schlitz 73 kann der mit dem Filter 31 verbundene Schlauch 36 von oben nach unten verschoben werden, wenn der Filter in die Aufnahme eingebracht wird, sodass der Schlauch radial von außen und von unten zu dem Filter geführt ist.

Nach dem Filter 31 wird der Schlauch 36 über zweite in der Abdeckung 9 vorgesehene Aussparungen 75, 79, 81, 83, die im Wesentlichen spiegelbildlich zu den Aussparungen 15, 17, 19 angeordnet sind, zu dem Produktbeutel 33 geführt, der sich radial außerhalb von der Aufnahme 29 befindet.

In der Aussparung 81 ist eine zweite Schlauchklemme 34 vorgesehen. Ein zweiter lichtempfindlicher Sensor 85 befindet sich in der Aussparung 79.

Zum Betätigen der Klemmen 34 sind innerhalb der Abdeckung 9 jeweils zwei Stangen 21, 23 als Betätigungseinrichtung so durch die Abdeckung geführt, dass eines ihrer Enden geringfügig aus einer der Zwischenwand 3 gegenüberliegenden Seitenfläche 8 der Abdeckung 9 herausragt und das andere Ende sich in dem Bereich der die Klemme 34 aufnehmenden Vertiefung 17 befindet. Durch das Ausüben eines Drucks auf eines der aus der Seitenfläche 8 ragenden Enden kann somit die handelsübliche Klemme 34 geöffnet bzw. geschlossen werden. Gemäß der Ausführungsform sind die Schlauchklemmen 34 sowohl einzeln als auch pneumatisch betätigbar.

Nach der Bestückung des Einsatzes y kann der Einsatz y in die Systembox 89 des Rotors einer Zentrifuge eingesetzt werden. Dabei ruht die der Zwischenwand 3 gegenüberliegende Seitenfläche 8 der Abdeckung 9 an einem Stützteil 57 der Systembox 89, das in dem Bereich einer Nabe der Zentrifuge vorgesehen ist. Bei dem Stützteil 57 befindet sich außerdem ein stabförmiges Verriegelungselement 55, das an seiner radialen Außenseite einen Vorsprung 56 aufweist. Durch das Einsetzen des Einsatzes y gleitet die Seitenfläche 8 der Abdeckung 9 über den Vorsprung 56 und bewegt das Verriegelungselement 55 radial nach innen, bis die Seitenfläche 8 unter den Vorsprung 56 gerät, und das Verriegelungselement 55 in die ursprüngliche Lage zurückfedert, und somit eine Verschiebung des Einsatzes y nach oben verhindert. Der Einsatz y ist nun fest zwischen der Außenwand der Systembox 89 und dem Stützelement positioniert.

Gemäß dem Ausführungsbeispiel ist der Rotor der Zentrifuge für sechs Systemboxen 89 mit jeweils einem Einsatz y ausgelegt. Nach dem Einschieben y aller Einsätze y wird die Zentrifuge gestartet. Durch die Fliehkraft kommt es zu der gewünschten Trennung der Blutbestandteile. Da sich das durch eine Additivlösung verdünnte "buffy coat" in dem Blutbeutel 35 befindet, werden dessen leichtere Bestandteile radial innen verbleiben, während schwerere Bestandteile d.h. die roten Blutkörperchen sich außen sammeln.

Um den gewünschten Blutbestandteil - gemäß der Ausführungsform sind dies die Blutplättchen - in hoher Qualität d.h. ohne Beimengung anderer Blutzellen aus dem Blutbeutel zu fördern, wird nach der Auftrennung der Bestandteile mittels einem bekannten Druckkissen 61 ein leichter Druck auf den Blutbeutel ausgeübt, sodass nach dem Öffnen der Klemmen 34 die plättchenreiche Lösung beginnt, in den nach oben und radial nach innen geführten Schlauch 36 aufzusteigen. Durch den Schlauch 36 wird die plättchenreiche Lösung in den Leukozytenfilter 31 geleitet, in den sie radial von außen und unten eintritt.

In dem Leukozytenfilter 31 werden die unerwünschten Leukozyten, das sind die weißen Blutkörperchen, entfernt. Aufgrund der erfindungsgemäßen Anordnung des Schlauchs 36 mit dem Filter 31 findet die Filtration entgegen der Zentrifugalkraft bzw. Fliehkraft statt. Dadurch werden schwerere Blutbestandteile wie ungewollt mitgeförderte rote Blutkörperchen in einer radial außen liegenden Filtereingangskammer abgefangen.

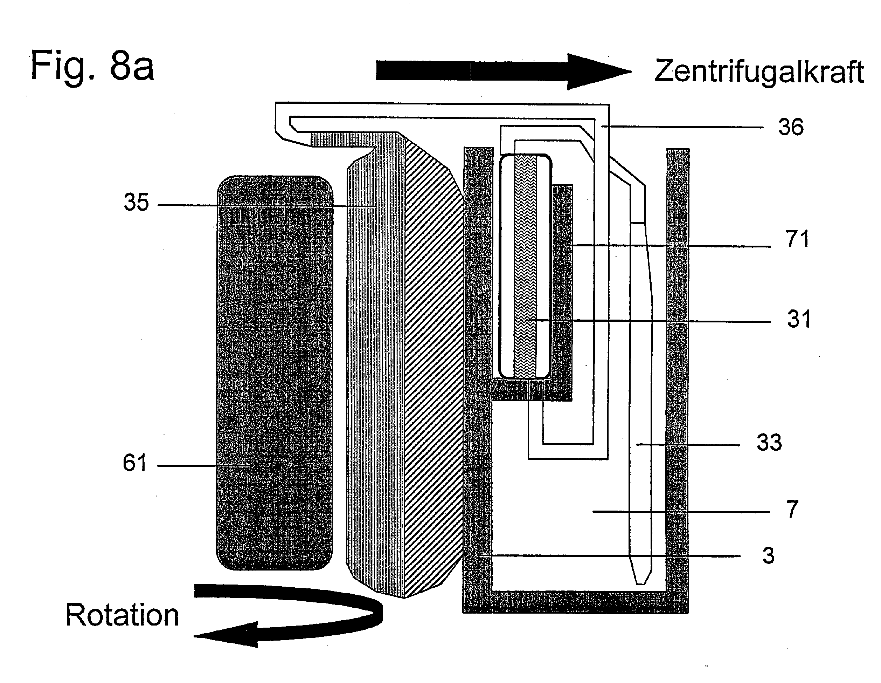

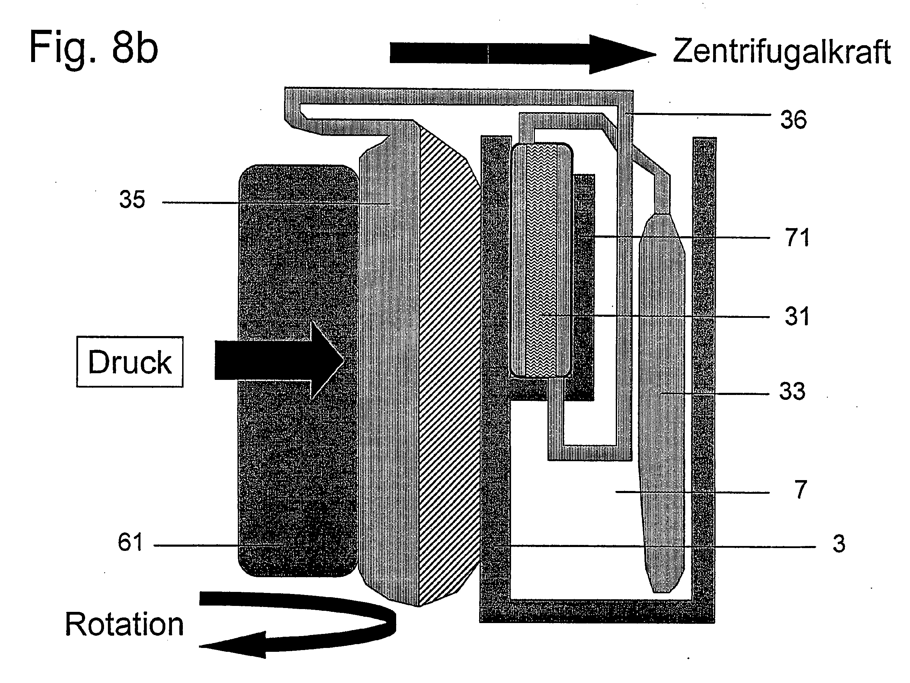

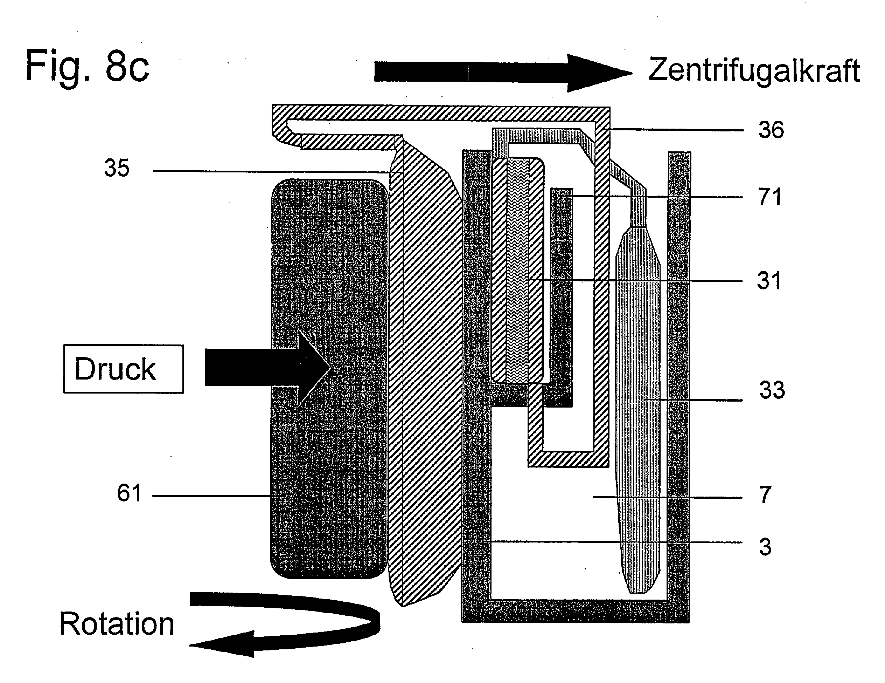

Nach dem Passieren des Leukozytenfilters 31 fließt die plättchenreiche Lösung schließlich weiter durch den Schlauch 36 in einen Produktbeutel 33, in dem sie gesammelt wird. Der Produktbeutel 33 ist vorzugsweise bereits als endgültiger Lagerbeutel für das Produkt ausgebildet. Der gesamte Vorgang ist in Fig. 8a bis 8c schematisch dargestellt.

Um eventuell vorhandene Luft in dem Filter zu entfernen, wird am Beginn der Produktüberleitung für eine bestimmte Volumenmenge die Fließgeschwindigkeit niedrig gehalten, und dadurch ermöglicht, dass sich der Filter zuverlässig und vollständig mit dem Blutprodukt füllt. Nach der Überleitung dieser bestimmten Volumenmenge wird mittels einer geeigneten Steuerung des Druckkissens, die Fördergeschwindigkeit für eine bestimmte zweite Volumenmenge erhöht. Während dieses zweite Volumen gefördert wird, besteht nur sehr geringe Gefahr, dass rote Blutkörperchen das Blutprodukt (hier das Thrombozytenkonzentrat) verunreinigen. Falls dies doch passiert, wird diese geringe Anzahl von roten Blutkörperchen, wegen der Führung des Schlauchs 36 von radial außen und unten in den Filter und der Wirkung der Zentrifugalkraft bzw. Fliehkraft, in dem unteren und äußeren Bereich des Filters gesammelt.

Nach dem Überleiten des zweiten Volumens wird der erste lichtempfindliche Sensor aktiviert und die Strömungsgeschwindigkeit des Blutprodukts in dem Schlauch 36 reduziert.

Erfasst der erste lichtempfindliche Sensor 25 einen vorbestimmten Anteil roter Blutkörperchen in der thrombozytenreichen Lösung, gibt er ein Signal ab, durch das die Strömungsgeschwindigkeit abermals reduziert wird. Außerdem wird der hinter dem Filter 31 angeordnete zweite lichtempfindliche Sensor 85 aktiviert.

In dieser Phase kann auch eine größere Anzahl roter Blutkörperchen in den Filter 31 eindringen und diesen sogar passieren, bis der zweite lichtempfindliche-Sensor 85 einen vorbestimmten Anteil roter Blutkörperchen in dem Blutprodukt erfasst und ein Signal zum Beenden des Zellgewinnungsvorgangs ausgibt. Durch dieses Signal werden die Schlauchklemmen 34 durch das Betätigen der Stange 23 geschlossen, sodass die roten Blutkörperchen in dem Filter zuverlässig von dem Thrombozytenkonzentrat im Produktbeutel getrennt sind. Die Betätigung der Stange erfolgt über einen in der Systembox 89 vorgesehenen Stellmechanismus.

Alternativ zur Beendigung durch den zweiten lichtempfindlichen Sensor 85, kann der Zellgewinnungsvorgang auch nach dem Verstreichen eines bestimmten Zeitraums nach dem Aktivieren des zweiten lichtempfindlichen Sensors 85 beendet werden.

In der Ausführungsform sind insgesamt sechs Einsätze in der Zentrifuge vorgesehen. Durch die vorangehend beschriebene Steuerung des Zellgewinnungsvorgangs in einem Einsatz y mittels Druckkissen 61 dem Öffnen und Schließen der Schlauchklemmen 34, und der Prozeßkontrolle mittels der zwei lichtempfindlichen Sensoren 25, 85 ist es möglich, die Zellgewinnung in den Einsätzen der übrigen Systemboxen 89 fortzusetzen da die beschriebene Prozeßsteuerung individuell für jede Kombination aus Einsatz und Systembox stattfindet.

Zur Übertragung der Steuer- und anderer elektrischer Signale ist auf dem Stützteil 57 der Systembox 89 eine elektrische Kontaktstelle in Form von einzelnen Kontaktpunkten 59 vorgesehen. An der Unterseite der Abdeckung 9 befinden sich den Kontaktpunkten 59 zugeordnete Kontaktflächen 27, die bei dem Einschieben des Einsatzes y in die Systembox mit den Kontaktpunkten 59 in Berührung geraten. Die Kontaktpunkte 59 sind zu diesem Zweck federnd gelagert.

Zur leichteren Handhabung einerseits und für den Fall, bei dem Blutbestandteile aufgrund von einer Beschädigung der Beutel 33, 35, des Schlauchs 36 oder des Filters 31 austreten, ist der Einsatz y von einer radial inneren Richtung in eine Auffangwanne eingeführt. In dem Fall einer Beschädigung wird der ausgetretene Blutbestandteil zum Großteil in der Auffangwanne gesammelt, sodass es nur zu geringfügigen Verschmutzungen der Systembox 89 oder des Rotors selbst kommt. Die Systembox 89 kann in einem derartigen Fall einfach aus dem Rotor ausgebaut werden.

Nach der Beendigung der Zellgewinnung wird jeder der Einsätze y entnommen, indem auf das Verriegelungselement 55 ein leichter Druck ausgeübt wird, um dieses radial nach innen zu bewegen.

Gleichzeitig werden die Einsätze y an Fingerlöchern der Auffangwanne erfasst und nach oben aus der Systembox 89 der Zentrifuge herausgehoben, um sofort durch neue, frisch bestückte Einsätze y ersetzt zu werden. Während der folgenden Zellgewinnung können die Blutbeutel 35 und Produktbeutel 33 aus den ausgetauschten Einsätzen y entnommen werden und diese wieder neu bestückt werden.

## Patentansprüche

1. Verfahren zur Zellgewinnung aus Blut oder aus Bluterzeugnissen mit folgenden Schritten:
(i) Einlegen eines Blutbeutels (35) mit einem Blutprodukt in einen Blutbeutelbereich (5) eines Einsatzes,
(ii) Einlegen eines mit dem Blutbeutel (35) verbundenen Schlauchs (36) in einen Produkt-Förderweg (36), der den Blutbeutel (35) mit einem Produktbeutel (33) verbindet, wobei entlang des Produkt-Förderwegs (36) ein erster Sensor (25) und ein zweiter Sensor (85) positioniert sind,
(iii) Einsetzen des Einsatzes in einen Rotor einer Zentrifuge,
(iv) Rotieren der Zentrifuge zum Auftrennen der einzelnen Blutbestandteile,
(v) Fördern einer vorbestimmten Menge eines Blutbestandteils entlang des Produkt-Förderwegs (36) mit einer ersten Strömungsgeschwindigkeit,
(vi) Erfassen der Zusammensetzung des geförderten Blutbestandteils durch den ersten Sensor (25), **dadurch gekennzeichnet, dass** der zweite Sensor (85) nach einer Abgabe eines einer vorbestimmten Zusammensetzung des Blutbestandteils entsprechenden Signals durch den ersten Sensor (25) ebenfalls die Zusammensetzung des geförderten Blutbestandteils erfasst, und das Fördern und das Rotieren beendet werden, wenn der zweite Sensor (85) die vorbestimmte Zusammensetzung erfasst und ein entsprechendes End-Signal abgibt, oder wenn eine vorbestimmte Zeit verstrichen ist.

2. Verfahren zur Zellgewinnung nach Anspruch 1, wobei vor dem Schritt (v) der Blutbestandteil mit einer reduzierten Anfangsgeschwindigkeit gefördert wird, um einen in dem Produkt-Förderweg (36) zwischen dem ersten Sensor (25) und dem zweiten Sensor (85) vorgesehenen Filter (31) zu entlüften, oder
wobei das Produkt radial von außen und unten zu dem Filter (31) gefördert wird und die Filtration gegen die Fliehkraft stattfindet.

3. Verfahren zur Zellgewinnung nach Anspruch 1 bis 2, wobei vor dem Fördern mit der ersten Strömungsgeschwindigkeit bzw. mit der reduzierten Anfangsgeschwindigkeit eine auf dem Schlauch (36) vorgesehene Schlauchklemme (34) durch eine in dem Einsatz vorgesehene Betätigungseinrichtung (21, 23) geöffnet wird.

4. Verfahren zur Zellgewinnung nach Anspruch 1 bis 3, wobei eine erste Schlauchklemme (34) zwischen dem ersten Sensor (25) und dem Filter (31) vorgesehen ist, und/oder eine zweite Schlauchklemme (34) zwischen dem zweiten Sensor (85) und dem Produktbeutel (33) bereitgestellt ist.

5. Verfahren zur Zellgewinnung nach Anspruch 4, wobei die erste Schlauchklemme (34) und/oder die zweite Schlauchklemme (34) bei dem Beginn der Förderung geöffnet und bei dem Ende der Förderung durch die Betätigungseinrichtung (21, 23) geschlossen werden.

6. Verfahren zur Zellgewinnung nach einem der Ansprüche 1 bis 5, wobei die ersten und zweiten Sensoren (25, 26) optische Sensoren sind, und die vorbestimmte Zusammensetzung des geförderten Blutbestandteils einen bestimmten Anteil roter Blutkörperchen aufweist.

7. Verfahren zur Zellgewinnung nach einem der Ansprüche 1 bis 6, wobei zur Anpassung der Strömungsgeschwindigkeit mittels eines bei dem Rotor vorgesehenen Druckelements (61) ein radial nach außen wirkender Druck auf den Blutbeutel (35) ausgeübt wird.

8. Verfahren nach einem der Ansprüche 1 bis 7, wobei der zweite Sensor (85) erst nach der Abgabe des der bestimmten Zusammensetzung entsprechenden Signals durch den ersten Sensor (25) aktiviert wird.

9. Verfahren nach einem der Ansprüche 1 bis 8, wobei die erste Strömungsgeschwindigkeit nach einer vorbestimmten Zeit oder bei Erreichung eines vorbestimmten Fördervolumens reduziert wird, und der Blutbestandteil mit der reduzierten Strömungsgeschwindigkeit weiter gefördert wird, und der erste Sensor die Zusammensetzung des Blutbestandteils nach der Reduzierung der Strömungsgeschwindigkeit erfasst.

10. Einsatz zum Einsatz in einer Zentrifuge zur Zellgewinnung aus Blut oder aus Bluterzeugnissen mit: einem Blutbeutelbereich (5) zum Einlegen eines Blutbeutels in einen Produktförderweg (36), der den Blutbeutel (35) mit einem Produktbeutel (33) verbindet, wobei entlang des Produkt-Förderwegs (36) ein erster Sensor (25) und ein zweiter Sensor (85) positioniert sind, **dadurch gekennzeichnet, dass** der erste Sensor (25) und der zweite Sensor (85) in Reihe geschaltet sind, und der zweite Sensor (85) aktivierbar ist, nach einer Abgabe eines, einer vorbestimmten Zusammensetzung eines in dem Produkt-Förderweg geförderten Blutbestandteils entsprechenden Signals durch den ersten Sensor (25) die Zusammensetzung des geförderten Blutbestandteils zu erfassen, und das Fördern beendet wird, wenn der zweite Sensor (85) die vorbestimmte Zusammensetzung erfasst und ein entsprechendes End-Signal abgibt, oder wenn eine vorbestimmte Zeit verstrichen ist.

11. Einsatz nach Anspruch 10, wobei zwischen dem ersten Sensor (25) und dem zweiten Sensor (85) ein Filter (31) vorgesehen ist, oder
wobei der Produkt-Förderweg radial von aussen und unten zu dem Filter (31) führt, um eine Filtration gegen die Fliehkraft zu ermöglichen.

12. Einsatz nach Anspruch 10 bis 11, wobei in dem Produkt-Förderweg ein Schlauch (36) vorgesehen ist, auf dem eine Schlauchklemme (34) bereitgestellt ist, die durch eine in dem Einsatz vorgesehene Betätigungseinrichtung (21 , 23) betätigbar ist.

13. Einsatz nach Anspruch 10 bis 12, wobei eine erste Schlauchklemme (34) zwischen dem ersten Sensor (25) und dem Filter (31) vorgesehen ist, und/oder eine zweite Schlauchklemme (34) zwischen dem zweiten Sensor (85) und dem Produktbeutel (33) vorgesehen ist.

14. Einsatz nach einem der Ansprüche 10 bis 13, wobei die ersten und zweiten Sensoren (25, 26) optische Sensoren sind.

15. Zentrifuge mit Einsatz nach einem der Ansprüche 10 bis 14, wobei zur Anpassung der Strömungsgeschwindigkeit ein bei dem Rotor vorgesehenes Druckelement (61) zur Ausübung eines radial nach außen wirkenden Drucks auf den Blutbeutel (35) vorgesehen ist.

## Claims

1. Method for cell separation from blood or from blood products comprising the following steps:
(i) inserting a blood bag (35) containing a blood product into a blood bag section (5) of a cassette,
(ii) inserting a tube (36) connected to the blood bag (35) into a product transport path (36), which connects the blood bag (35) to a product bag (33), wherein a first sensor (25) and a second sensor (85) are positioned along the product transport path (36),
(iii) inserting the cassette into a rotor of a centrifuge,
(iv) rotating the centrifuge in order to separate the individual blood components,
(v) transporting a predetermined quantity of a blood component along the product transport path (36) at a first flow speed,
(vi) detecting the composition of the transported blood component by means of the first sensor (25),
**characterized in that**
after the first sensor (25) has output a signal corresponding to a predetermined composition of the blood component, also the second sensor (85) detects the composition of the transported blood component, and the transporting is terminated, when the second sensor (85) detects the predetermined composition and outputs a corresponding end signal, or after a predetermined period of time has elapsed.

2. Method for cell separation according to claim 1, wherein the blood component is transported at a reduced initial speed before step (v) in order to ventilate a filter (31) provided in the product transport path (36) between the first sensor (25) and the second sensor (85), or wherein the product is transported to the filter (31) radially from the outside and from below and the filtration takes place against the centrifugal force.

3. Method for cell separation according to claims 1 to 2, wherein a tube clamp (34) provided on the tube (36) is opened by means of an operating device (21, 23) provided in the cassette, before transporting at a first flow speed or respectively at a reduced initial speed is started.

4. Method for cell separation according to claims 1 to 3, wherein a first tube clamp (34) is provided between the first sensor (25) and the filter (31) and/or a second tube clamp (34) is provided between the second sensor (85) and the product bag (33).

5. Method for cell separation according to claim 4, wherein the first tube clamp (34) and/or the second tube clamp (34) is/are opened, when transporting is started, and closed, when transporting is terminated, by means of the operating device (21, 23).

6. Method for cell separation according to one of the claims 1 to 5, wherein the first and the second sensors (25, 26) are optical sensors, and the predetermined composition of the transported blood component shows a certain proportion of red blood cells.

7. Method for cell separation according to one of the claims 1 to 6, wherein for adjusting the flow speed a pressure acting radially outward is exerted onto the blood bag (35) by means of a pressing element (61) provided at the rotor.

8. Method according to one of the claims 1 to 7, wherein the second sensor (85) is only activated after the first sensor (25) has output a signal indicating the predetermined composition.

9. Method according to one of the claims 1 to 8, wherein the first flow speed is reduced after a predetermined period of time, and the blood component is further transported at a reduced flow speed, and the first sensor detects the composition of the blood component after the flow speed has been reduced.

10. Cassette to be inserted into a centrifuge for the cell separation from blood or from blood products having:
a blood bag section (5) for inserting a blood bag,
a product transport path (36), which connects the blood bag (35) to a product bag (33), wherein a first sensor (25) and a second sensor (85) are positioned along the product transport path (36),
**characterized in that**
the first sensor (25) and the second sensor (85) are provided in series,
and the second sensor (85) can be activated to detect the composition of the transported blood component after the first sensor (25) has output a signal indicating a predetermined composition of the blood component transported in the product transport path, and the transporting is terminated when the second sensor (85) records the predetermined composition and outputs a corresponding end signal, or after a predetermined period of time has elapsed.

11. Cassette according to claim 10, wherein a filter (31) is provided between the first sensor (25) and the second sensor (85), or wherein the product transport path leads to the filter (31) radially from the outside and from below to enable filtration against the centrifugal force.

12. Cassette according to claims 10 to 11, wherein a tube (36) on which a tube clamp (34) is provided is disposed in the product transport path (36), said tube clamp (34) being operable by means of an operating device (21, 23) provided in the cassette.

13. Cassette according to claims 10 to 12, wherein a first tube clamp (34) is provided between the first sensor (25) and the filter (31) and/or a second tube clamp (34) is provided between the second sensor (85) and the product bag (33).

14. Cassette according to one of the claims 10 to 13, wherein the first and the second sensors (25, 85) are optical sensors.

15. Centrifuge having a cassette according to one of the claims 10 to 14, wherein, for adjusting the flow speed, a pressing element (61) provided at the rotor is provided for exerting a pressure acting radially outward onto the blood bag (35).

## Revendications

1. Procédé d'obtention de cellules à partir du sang ou de produits sanguins, comportant les étapes suivantes :
(i) mise en place d'une poche de sang (35) avec un produit sanguin dans une zone pour poche de sang (5) d'une garniture,
(ii) mise en place d'un tuyau (36) relié à la poche de sang (35) dans une voie de refoulement de produit (36) qui relie la poche de sang (35) à une poche de produit (33), un premier capteur (25) et un deuxième capteur (85) étant positionnés le long de la voie de refoulement de produit (36),
(iii) mise en place de la garniture dans un rotor d'une centrifugeuse,
(iv) rotation de la centrifugeuse afin de séparer les différents composants sanguins,
(v) refoulement à une première vitesse d'écoulement d'une quantité prédéfinie d'un composant sanguin le long de la voie de refoulement de produit (36),
(vi) détermination de la composition du composant sanguin refoulé par le premier capteur (25),
**caractérisé en ce que** le deuxième capteur (85), après délivrance par le premier capteur (25) d'un signal correspondant à une composition prédéfinie du composant sanguin, détermine lui aussi la composition du composant sanguin refoulé, et **en ce que** le refoulement et la rotation sont arrêtés quand le deuxième capteur (85) détermine la composition prédéfinie et émet un signal terminal correspondant, ou quand un temps prédéfini s'est écoulé.

2. Procédé d'obtention de cellules selon la revendication 1, où, préalablement à l'étape (v), le composant sanguin est refoulé à une vitesse initiale réduite, pour purger l'air d'un filtre (31) prévu dans la voie de refoulement de produit (36) entre le premier capteur (25) et le deuxième capteur (85), ou bien
où le produit est refoulé radialement depuis l'extérieur et par en dessous vers le filtre (31), et où la filtration est effectuée contre la force centrifuge.

3. Procédé d'obtention de cellules selon les revendications 1 et 2, où, préalablement au refoulement à la première vitesse d'écoulement ou à la vitesse initiale réduite, un collier de serrage (34) prévu sur le tuyau (36) est ouvert par un dispositif d'actionnement (21, 23) prévu dans la garniture.

4. Procédé d'obtention de cellules selon la revendication 1 à 3, où un premier collier de serrage (34) est prévu entre le premier capteur (25) et le filtre (31), et/ou où un deuxième collier de serrage (34) est monté entre le deuxième capteur (85) et la poche de produit (33).

5. Procédé d'obtention de cellules selon la revendication 4, où le premier collier de serrage (34) et/ou le deuxième collier de serrage (34) sont ouverts par le dispositif d'actionnement (21, 23) au début du refoulement et fermés à la fin du refoulement.

6. Procédé d'obtention de cellules selon l'une des revendications 1 à 5, où les premier et deuxième capteurs (25, 26) sont des capteurs optiques, et où la composition prédéfinie du composant sanguin refoulé comprend une teneur définie en globules rouges.

7. Procédé d'obtention de cellules selon l'une des revendications 1 à 6, où, pour ajuster la vitesse d'écoulement, une pression s'exerçant radialement vers l'extérieur est appliquée sur la poche de sang (35) au moyen d'un élément de pression (61) prévu pour le rotor.

8. Procédé selon l'une des revendications 1 à 7, où le deuxième capteur (85) n'est activé qu'après émission par le premier capteur (25) du signal correspondant à la composition prédéfinie.

9. Procédé selon l'une des revendications 1 à 8, où la première vitesse d'écoulement est réduite après un temps prédéfini ou lorsqu'un débit de refoulement prédéfini est atteint, et où le composant sanguin continue à être refoulé à la vitesse d'écoulement réduite, et où le premier capteur détermine la composition du composant sanguin après réduction de la vitesse d'écoulement.

10. Garniture destinée à être utilisée dans une centrifugeuse pour l'obtention de cellules à partir du sang ou de produits sanguins, avec : une zone pour poche de sang (5) pour la mise en place d'une poche de sang dans une voie de refoulement de produit (36) reliant la poche de sang (35) à une poche de produit (33), un premier capteur (25) et un deuxième capteur (85) étant positionnés le long de la voie de refoulement de produit (36), **caractérisée en ce que** le premier capteur (25) et le deuxième capteur (85) sont montés en ligne, **en ce que** le deuxième capteur (85) peut être activé après émission par le premier capteur (25) d'un signal correspondant à une composition prédéfinie d'un composant sanguin refoulé dans la voie de refoulement de produit, pour déterminer la composition du composant sanguin refoulé, et **en ce que** le refoulement est arrêté quand le deuxième capteur (85) détermine la composition prédéfinie et émet un signal terminal correspondant, ou après l'écoulement d'un temps prédéfini.

11. Garniture selon la revendication 10, où un filtre (31) est prévu entre le premier capteur (25) et le deuxième capteur (85), ou bien
où la voie de refoulement de produit mène radialement depuis l'extérieur et par en dessous vers le filtre (31), pour permettre une filtration contre la force centrifuge.

12. Garniture selon les revendications 10 et 11, où un tuyau (36) est prévu dans la voie de refoulement de produit, sur lequel est monté un collier de serrage (34) pouvant être actionné par un dispositif d'actionnement (21, 23) prévu dans la garniture.

13. Garniture selon les revendications 10 à 12, où un premier collier de serrage (34) est prévu entre le premier capteur (25) et le filtre (31), et/ou où un deuxième collier de serrage (34) est prévu entre le deuxième capteur (85) et la poche de produit (33).

14. Garniture selon l'une des revendications 10 à 13, où les premier et deuxième capteurs (25, 26) sont des capteurs optiques.

15. Centrifugeuse avec une garniture selon l'une des revendications 10 à 14, où, pour ajuster la vitesse d'écoulement, il est prévu un élément de pression (61) pour le rotor, pour l'application d'une pression s'exerçant radialement vers l'extérieur sur la poche de sang (35).
